# EUROPEAN PATENT SPECIFICATION

(11) **EP 2 127 592 B1**
(45) Date of publication and mention of the grant of the patent: **19.12.2012**
(21) Application number: 08711657.0
(22) Date of filing: 20.02.2008
(51) Int. Cl.: H04N 5/235, A61B 1/04, A61B 1/06, H04N 5/225, A61B 5/07, A61B 1/00

(54) **INTRASUBJECT INTRODUCTION SYSTEM**
SYSTEM ZUR EINFÜHRUNG IN EIN SUBJEKT
SYSTÈME D'INTRODUCTION DANS UN SUJET

(30) Priority: 22.02.2007 JP 2007042449
(43) Date of publication of application: 02.12.2009
(73) Proprietor: Olympus Medical Systems Corp., Tokyo 151-0072 (JP); Olympus Corporation, Tokyo 151-0072 (JP)
(72) Inventor: SATO, Ryoji, Tokyo 151-0072 (JP); KAWANO, Hironao, Tokyo 151-0072 (JP); ORIHARA, Tatsuya, Tokyo 151-0072 (JP); MORI, Takeshi, Tokyo 151-0072 (JP)
(74) Representative: von Hellfeld, Axel
(86) International application number: PCT/JP2008/052851
(87) International publication number: WO 2008/102803

(56) References cited:
- WO-A2-02/073507
- WO-A2-02/073507
- JP-A- 2003 210 392
- JP-A- 2005 198 794
- JP-A- 2006 136 453
- JP-A- 2006 288 832
- JP-A- 2006 334 115
- JP-A- 2007 282 965
- US-A1- 2006 231 749

## Description

### TECHNICAL FIELD

The present invention relates to a body-insertable apparatus system that takes in-vivo images of a subject.

### BACKGROUND ART

Swallowable capsule endoscopes have been developed recently in the field of endoscopy. A capsule endoscope has image taking and wireless-communication functions. After being swallowed for observation (examination) by a subject, the capsule endoscope moves through the body cavity, for example, the internal organs, such as the stomach and the small intestine, by peristalsis and sequentially takes in-vivo images of the body cavity until the capsule endoscope is naturally excreted from the body.

In the field of endoscopy, a narrow-band observation method has been proposed that uses an illuminating light whose spectral characteristic has a bandwidth narrower than the bandwidth of an illuminating light that is used in a conventional RGB frame sequential method (see, for example, Patent Document 1). In the narrow-band observation method, by emitting a light covering two narrow bandwidths of blue light and of green light that are easily absorbed by hemoglobin in blood, blood capillaries in the surface layer of a membrana mucosa and the fine pattern of the membrana mucosa are made conspicuous when displayed. This leads to an early detection of a bleeding site or a tumor site, which is a detection-target site.

Patent Document 1: Japanese Patent Application Laid-open No. 2002-95635
JP 2006-334115 A may be construed to disclose an endoscopic diagnosis support device for supporting an endoscopic diagnosis made based on an endoscopic image imaged by the endoscopic observation device identifies an image region including the site of bleeding by calculating color tones from a color signal of each of a plurality of image regions acquired by dividing the endoscopic image and by deciding the difference among a plurality of the image regions from the calculated color tone of each image region.

### DISCLOSURE OF INVENTION

### PROBLEM TO BE SOLVED BY THE INVENTION

Because blood capillaries in the surface layer of a membrana mucosa and the fine pattern of the membrana mucosa can be made dark when using the narrow-band observation method, the blood capillaries in the surface layer of the membrana mucosa and the fine pattern of the membrana mucosa are made conspicuous. When taking images with a capsule endoscope whose light emitting unit emits a limited amount of light or taking images of a quickly moving object, some areas are displayed dark for the following causes: absorption by hemoglobin in blood, the viewing direction is in a direction of a lumen in which there is nothing present, or the amount of light is insufficient. However, the cause for the dark-displayed area cannot be distinguished. This leads to the problem that detection-target sites, such as bleeding sites and tumor sites, cannot be accurately detected.

The present invention addresses the above problem. An object of the present invention is to provide a body-insertable apparatus system that can detect a detection-target site in an image that is taken by a body-insertable apparatus.

### MEANS FOR SOLVING PROBLEM

A body-insertable apparatus system according to one aspect of the present invention that takes an in-vivo image of a subject includes: a body-insertable apparatus that includes a light emitting unit that emits at least a light having a low-reflectance wavelength with a low reflectance and a light having a high-reflectance wavelength with a high reflectance to a detection-target site that has predetermined optical characteristics corresponding to a detection target; and an imaging unit that receives the light having the low-reflectance wavelength and the light having the high-reflectance wavelength to take the in-vivo image; and a detector that detects the detection-target site of a detection-target area in the subject based on an amount of the light having the high-reflectance wavelength and an amount of the light having the low-reflectance wavelength of an area of the in-vivo image, the area of the in-vivo image corresponding to the detection-target area.

In the body-insertable apparatus system according to, the detector may extract an area where the amount of the light having the high-reflectance wavelength is equal to or above a threshold from the in-vivo image as an inclusion area that may include the detection-target site, and may detect the detection-target site based on the amount of the light having the low-reflectance wavelength in the extracted inclusion area.

In the body-insertable apparatus system according to the detector may extract an area where the amount of the light having the high-reflectance wavelength is equal to or above the threshold from an image of the high-reflectance wavelength as the inclusion area and may detect the detection-target site based on the amount of the light having the low-reflectance wavelength in an area of an image of the low-reflectance wavelength, the area of the image corresponding to the inclusion area.

In the body-insertable apparatus system according , the threshold may be set based on a reflectance of the light having the low-reflectance wavelength and a reflectance of the light having the high-reflectance wavelength with respect to the detection-target site.

In the body-insertable apparatus system according to, the detection-target site may be a bleeding site.

In the body-insertable apparatus system according to, the low-reflectance wavelength may be in a range of 415 to 580 nm (nanometers), and the high-reflectance wavelength may be in a range of 615 to 635 nm.

The body-insertable apparatus system according to the may further include an image processing unit that processes the in-vivo image; and an image display unit that displays information that contains the in-vivo image processed by the image processing unit, wherein the image processing unit may perform an enhancement process for enhancing the detection-target site.

In the body-insertable apparatus system according to, the light emitting unit and the imaging unit may be provided on a side surface of the body-insertable apparatus.

In the body-insertable apparatus system according to, the body-insertable apparatus may include at least two imaging units and two light emitting units, and the two imaging units take images in opposite directions.

In the body-insertable apparatus system according to, the light emitting unit may include at least a light emitting device that emits the light having the low-reflectance wavelength; and a light emitting device that emits the light having the high-reflectance wavelength.

In the body-insertable apparatus system according to, the light emitting devices may be LEDs.

In the body-insertable apparatus system according to the present invention, the body-insertable apparatus includes may further include a drive unit that supplies electric power to each of the light emitting devices to cause each of the light emitting devices to emit the light; and a control unit that controls an amount of the electric power supplied by the drive unit to each of the light-emitting devices.

In the body-insertable apparatus system according to, the drive unit may include a current amount adjusting unit that adjusts an amount of current to be supplied to the light emitting devices, and the control unit may control the current amount adjusting unit to control the amount of current to be supplied to each of the light emitting devices.

In the body-insertable apparatus system according to, the drive unit may include a power supply selecting unit that can select whether to supply electric power to each of the light emitting devices, and the control unit may control the power supply selecting unit to control the amount of electric power to be supplied to each of the light emitting devices.

In the body-insertable apparatus system according to, the power supply selecting unit may select whether to supply electric power to each of the light emitting devices, and may select the amount of electric power to be supplied to each of the light emitting devices.

In the body-insertable apparatus system according to the present invention, the power supply selecting unit may change a period during which electric power is supplied to each of the light emitting devices.

In the body-insertable apparatus system according to , the power supply selecting unit may supply electric power to each of the light emitting devices in a time-division manner.

In the body-insertable apparatus system according to , the control unit may control the amount of electric power to be supplied to each of the light emitting devices based on a reflectance of the light having the low-reflectance wavelength with respect to the detection-target site and a reflectance of the light having the high-reflectance wavelength with respect to the detection-target site.

In the body-insertable apparatus system according to , the control unit may control the amount of electric power to be supplied to each of the light emitting devices based on a value of white balance.

In the body-insertable apparatus system according to, the body-insertable apparatus may further include a permanent magnet for guidance, and the body-insertable apparatus system may further include: a guidance magnetic field generating unit that generates a guidance magnetic field that is applied to the permanent magnet; and a guidance magnetic field direction control unit that controls a direction of the guidance magnetic field.

### EFFECT OF THE INVENTION

In the present invention, an image is taken by applying a light having a high-reflectance wavelength with a high reflectance with respect to a detection-target site and a light having a low-reflectance wavelength with a low reflectance. Based on an amount of the light having the high-reflectance wavelength and an amount of the light having the low-reflectance wavelength in an area of the image, which corresponds to a detection-target area of a subject, on which detection on whether there is a detection-target site is performed, a detection-target site of the detection-target area is detected. Thus, the detection-target site in the image can be detected.

### BRIEF DESCRIPTION OF DRAWINGS

FIG. 1 is a diagram of an entire configuration of a body-insertable apparatus system according to a first embodiment.
FIG. 2 is a schematic diagram for explaining a configuration of a capsule endoscope shown in FIG. 1.
FIG. 3 is a block diagram of a configuration of a workstation shown in FIG. 1.
FIG. 4 is a diagram for explaining reflection of lights that are applied to a subject shown in FIG. 1.
FIG. 5 is a graph of reflectance of the lights that are applied to the subject shown in FIG. 1.
FIG. 6 is a flowchart of a procedure of a detecting process in the body-insertable apparatus system shown in FIG. 1.
FIG. 7 is a flowchart of a procedure of the detecting process shown in FIG. 6.
FIG. 8 is a diagram for explaining the capsule endoscope shown in FIG. 1 being inserted into the subject.
FIG. 9 is a diagram of an example of images that are taken by the capsule endoscope shown in FIG. 1.
FIG. 10 is a graph of reflectance of the lights applied to the subject shown in FIG. 1.
FIG. 11 is a schematic diagram for explaining another configuration of the capsule endoscope shown in FIG. 1.
FIG. 12 is a block diagram of a configuration of a receiving apparatus shown in FIG. 1.
FIG. 13 is a block diagram of another configuration of the capsule endoscope shown in FIG. 1.
FIG. 14 is a schematic diagram for explaining another configuration of the capsule endoscope shown in FIG. 1.
FIG. 15 is a block diagram of a configuration of a capsule endoscope according to a second embodiment.
FIG. 16 is a diagram for explaining the capsule endoscope that is attached with a cap shown in FIG. 15.
FIG. 17 is a block diagram of a main part of the capsule endoscope shown in FIG. 15.
FIG. 18 is a block diagram of another example of the main part of the capsule endoscope shown in FIG. 15.
FIG. 19 is a block diagram of still another example of the main part of the capsule endoscope shown in FIG. 15.
FIG. 20 is a timing chart representing how switches shown in FIG. 19 are driven.
FIG. 21 is another timing chart representing how the switches shown in FIG. 19 are driven.
FIG. 22 is a block diagram of a configuration of a receiving apparatus according to the second embodiment.
FIG. 23 is a block diagram of a configuration of a workstation according to the second embodiment.
FIG. 24 is a schematic diagram of a configuration of a capsule endoscope according to a third embodiment.
FIG. 25 is a schematic diagram of a body-insertable apparatus system according to the third embodiment.
FIG. 26 is a diagram for explaining the body-insertable apparatus system shown in FIG. 25.
FIG. 27 is a diagram for explaining another example of the body-insertable apparatus systems according to the first to third embodiments.

### EXPLANATIONS OF LETTERS OR NUMERALS

- 1: Subject
- 2, 202: Receiving apparatus
- 3, 3a, 3b, 203, 303: Capsule endoscope
- 4, 204, 304: Workstation
- 5: Portable recording medium
- 6a to 6h: Receiving antenna
- 7: External device
- 20: Antenna selector
- 21: Receiving circuit
- 22: Signal processing circuit
- 23: Storage unit
- 24, 224: Control unit
- 25: Power supply unit
- 30, 30a: Housing
- 31: End transparent cover
- 31a: Transparent cover
- 32: Light emitter
- 33: Lens
- 34, 334: Imaging device
- 35: Processing circuit
- 35a, 235a: Control unit
- 35b: Light receiving circuit
- 35c: Light-emitter driver
- 36: Antenna
- 37: Battery
- 41, 241, 341: Control unit
- 42: Input unit
- 43: Storage unit
- 44: Position detector
- 45: Detector
- 46: Image processor
- 47: Output unit
- 205: Cap
- 206a to 206h: Antenna
- 240: Light amount adjuster
- 254: Resistance setting unit
- 250: LED driver
- 256: Current value setting unit
- 320a: Constant voltage driver
- 320b: Constant current driver
- 320, 321 to 32n: LED
- 2511 to 251n: Current value setting unit
- 2521 to 252n: Driver
- 2531 to 253n: Resistor
- 2551 to 255n: Switch
- 338: Permanent magnet
- 371X, 371Y, 371Z: Helmholtz coil unit
- 372X, 372Y, 372Z: Helmholtz coil driver
- 373: Rotating magnetic field control circuit
- 374: Input device

### BEST MODE(S) FOR CARRYING OUT THE INVENTION

Embodiments of the present invention will be described below with reference to the accompanying drawings, taking as an example a body-insertable apparatus system that uses the narrow-band observation method. The embodiments do not limit the invention. Identical portions are denoted by the same reference numerals in the drawings.

### (First embodiment)

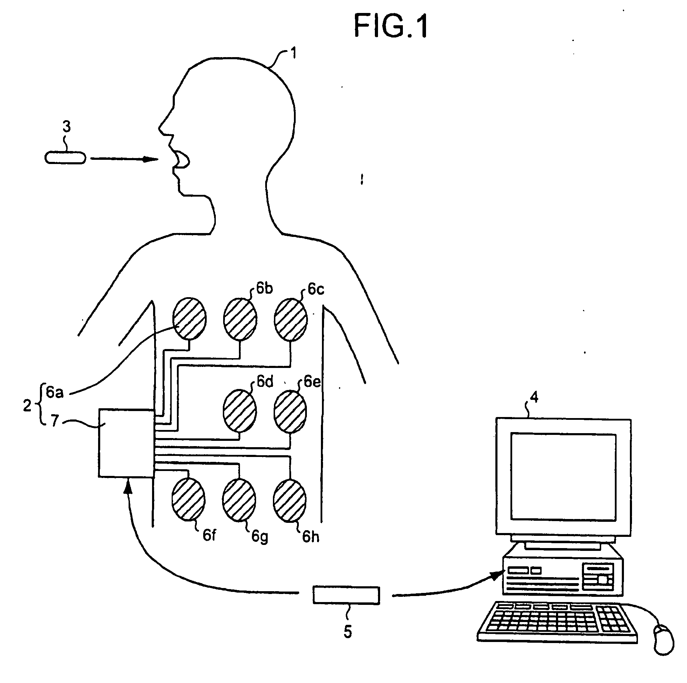

A first embodiment will be described below. FIG. 1 is a schematic diagram of an entire configuration of a body-insertable apparatus system according to the first embodiment. As shown in FIG. 1, the body-insertable apparatus system includes a receiving apparatus 2 that has a function of wireless reception; and a capsule endoscope 3 that is inserted into a subject 1, takes in-vivo images of the subject, and transmits the in-vivo images to the receiving apparatus 2 by wireless communications. The body-insertable apparatus system further includes a workstation 4 that processes information received and processed by the receiving apparatus 2, and that outputs and displays the in-vivo images; and a portable recording medium 5 for communicating information between the receiving apparatus 2 and the workstation 4. The receiving apparatus 2 includes receiving antennas 6a to 6h that receive wireless signals, which are transmitted from the capsule endoscope 3; and an external device 7 that performs a demodulating process on a wireless signal, which has the highest strength and is received by one of the receiving antennas, to acquire an in-vivo image of the subject 1. The portable recording medium 5 can be detachably attached to the external device 7 or the workstation 4. The portable recording medium 5 is configured to output or record information when being attached to the external device 7 or the workstation 4.

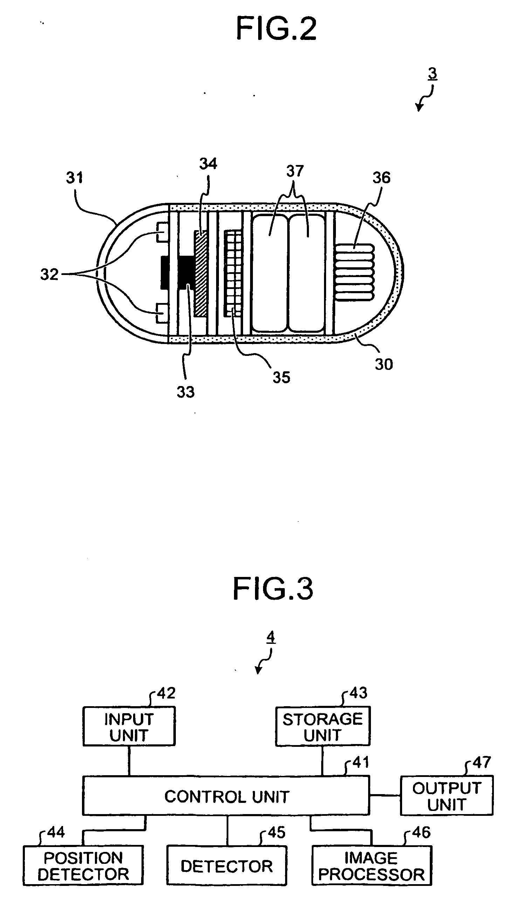

The capsule endoscope 3 shown in FIG. 1 will be described below with reference to FIG. 2. As shown in FIG. 2, the capsule endoscope 3 includes a light emitter 32 that emits a light having a wavelength in a predetermined bandwidth when taking an in-vivo image of the subject 1; a lens 33 that focuses the light, which has been emitted from the light emitter 32 and reflected on the interior of the subject 1; an image-taking device 34 that is embodied by, for example, a CCD, and that takes an in-vivo image of the subject by receiving the light focused by the lens 33; a processing circuit 35 that generates a wireless signal corresponding to the image taken by the imaging device 34, and that controls driving of the light emitter 32, the imaging device 34, and an antenna 36; the antenna 36 that wirelessly transmits the wireless signal output from the processing circuit 35; and a battery 37 that supplies electric power to the light emitter 32, the imaging device 34, the processing circuit 35, and the antenna 36. The light emitted from the light emitters 32 is applied to the interior of the subject 1 through an end transparent cover 31 that is provided to an end portion of a housing 30. The light that is reflected on the interior of the subject 1 and incident on the capsule endoscope 3 through the end transparent cover 31 is focused by the lens 33. Thereafter, the light is received by the imaging device 34 and the amount of the received light is measured for every predetermined wavelength.

The light emitter 32 emits at least a light having a low-reflectance wavelength with a low reflectance and a light having a high-reflectance wavelength with a high reflectance to a detection-target site that has predetermined optical characteristics corresponding to a detection target. The light emitter 32 includes, for example, an LED that emits a white light. The detection-target site is often a bleeding site or a tumor site. The low-reflectance wavelength has any one of the wavelengths from 415 nm to 580 nm that are easily absorbed in hemoglobin of blood. The high-reflectance wavelength has any one of the wavelengths from 615 nm to 635 nm that are not easily absorbed in hemoglobin of blood. The imaging device 34 takes an in-vivo image of the subject 1 by receiving at least the light having the low-reflectance wavelength and the light having the high-reflectance wavelength. For example, the imaging device 34 can receive a blue light and a green light each having the low-reflectance wavelength as well as a red light having the high-reflectance wavelength.

The workstation shown in FIG. 1 will be described below. FIG. 3 is a block diagram of a configuration of the workstation shown in FIG. 1. As shown in FIG. 3, the workstation 4 includes a control unit 41 that controls units forming the workstation 4; an input unit 42 that input instruction information for processes to be performed by the workstation 4; a storage unit 43 that is embodied by, for example, a hard disk device, and that stores information including the image taken by the capsule endoscope 3; a position detector 44 that calculates a position and a direction of the capsule endoscope 3 based on intensities of wireless signals received by the receiving antennas 6a to 6h; a detector 45 that detects a detection-target site of a detection-target area in the subject 1 based on the image taken by the capsule endoscope 3; an image processor 46 that processes the image taken by the capsule endoscope 3; and an output unit 47 that is embodied by, for example, a display, and that outputs and displays the information including the image processed by the image processor 46. A detection-target area is an area where a detection whether the detection-target site exists or not is performed. The detector 45 detects a detection-target site of such a detection-target area in the subject 1 from the image taken by the imaging device 34 based on an amount of the light having the high-reflectance wavelength and an amount of the light having the low-reflectance wavelength. The image processor 46 performs the process for making the detection-target site conspicuous based on a result of detecting the detection-target site.

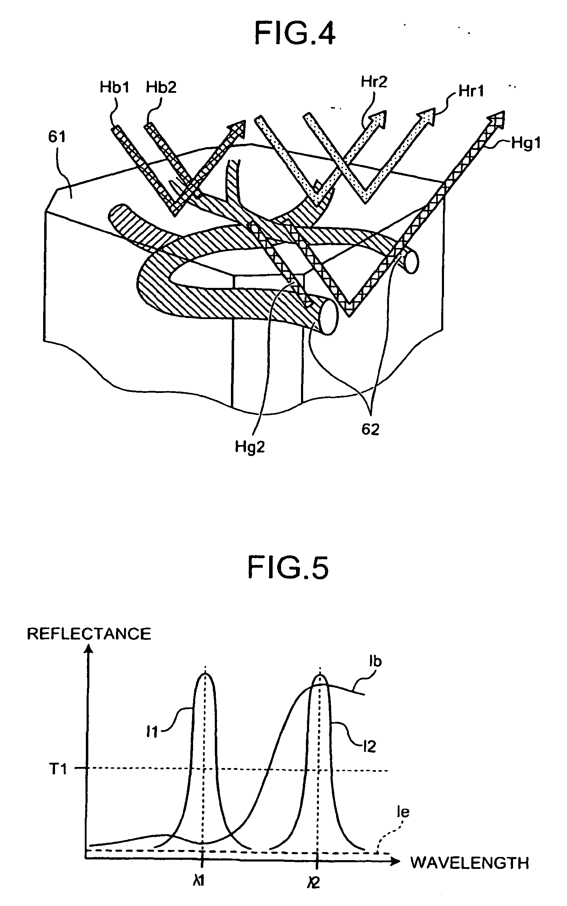

The light having the low-reflectance wavelength and the light having the high-reflectance wavelength emitted by the light emitter 32 of the capsule endoscope 3 will be described below with reference to FIG. 4. When a blue light in the bandwidth from 415 nm to 445 nm included in the bandwidth from 415 nm to 580 nm that corresponds to the low-reflectance wavelength is incident on an area without a bleeding site, most of the blue light is reflected on the body surface, such as a skin. 61, as indicated by an incident light Hb1 shown in FIG. 4. In contrast, when the blue light is incident on a blood vessel, the blue light is absorbed in hemoglobin in a blood vessel 62 and is hardly reflected, as indicated by an incident light Hb2. A green light in the bandwidth from 530 nm to 550 nm included in the bandwidth from 415 nm to 580 nm can enter a deep portion deeper than the layer where the blue light can enter. When the green light is incident on an area without a bleeding site, most of the green light is reflected at the inside of the skin 61 as indicated by an incident light Hg1 shown in FIG. 4. In contrast, when the green light is incident on a blood vessel, the green light is absorbed in the blood vessel 62 and is hardly reflected as indicated by an incident light Hg2. By processing an image of the blue light or the green light, which is easily absorbed in hemoglobin in the blood vessel 62, i.e., the low-reflectance wavelength, the image processor 46 obtains an image in which blood capillaries in a surface layer of a membrana mucosa and the fine pattern of the membrana mucosa are made conspicuous and a bleeding site or a main site, which is the detection-target site, is enhanced. In contrast, most of a red light within the bandwidth from 615 nm to 635 nm that corresponds to the high-reflectance wavelength is reflected on a body surface, such as the skin 61, as indicated by an incident light Hr1 and an incident light Hr2, regardless whether there is a bleeding site or not.

Reflectances of the high-reflectance wavelength and the low-reflectance wavelength will be described below with reference to FIG. 5. A curve 11 shown in FIG. 5 represents a characteristic of the light having a low-reflectance wavelength λ1, which is received by the imaging device 34. A curve 12 shown in FIG. 5 represents a characteristic of the light having a high-reflectance wavelength λ2, which is received by the imaging device 34. A curve lb represents a reflectance of each wavelength obtained at a breeding site. A straight line le represents a reflectance obtained when a light of each wavelength is emitted in the direction of a lumen where nothing is present. As indicated by the curve lb, when a light is emitted to a bleeding site, the light having the low-reflectance wavelength λ1 is absorbed in the bleeding site and therefore represents a low reflectance. In contrast, the light having the high-reflectance wavelength λ2 is reflected, even when there is a bleeding site, and therefore represents a high reflectance. Thus, when the amount of the light having the high-reflectance wavelength λ2, which is received by the imaging device 34, is sufficiently larger than the amount of the light having the low-reflectance wavelength λ1, which is received by the imaging device 34, even in consideration for diffuse reflection and measurement errors, it is considered that there is the detection-target site in the area on which the light is reflected. When a light is emitted toward an area where nothing is present, the light does not return. Therefore, the reflectance is approximately 0 at every wavelength as the straight line le shown in FIG. 5 represents.

The detector 45 detects a detection-target site based on a predetermined threshold that is set based on the reflectance of the light having the low-reflectance wavelength and the reflectance of the light having the high-reflectance wavelength with respect to the detection-target site. To set the predetermined threshold, first, a reflectance T1, from which it is determined that a detection-target site is highly likely present, is set in consideration for the reflectance of the light having the low-reflectance wavelength λ1 of the curve lb, the reflectance of the light having the high-reflectance wavelength λ2 of the curve lb, diffuse reflection, and measurement errors. Based on the reflectance T1 and an output value that is required by the light emitter 32 to emit the high-reflectance wavelength λ2, an output value that corresponds to an amount of the light having the high-reflectance wavelength λ2 and is thought to be received by the imaging device 34 is obtained, from the light having the high-reflectance wavelength λ2 and is emitted by the light emitter 32. Based on the obtained output value, and by taking measurement errors into consideration, the threshold is set.

The detector 45 extracts an area, in which the amount of the right having the high-reflectance wavelength is equal to or above the predetermined threshold, from the image as an inclusion area that may include a detection-target site. Based on the amount of the light having the low-reflectance wavelength in the extracted inclusion area, the detector 45 detects the detection-target site. For example, the detector 45 extracts an area, in which the amount of the light having the high-reflectance wavelength is equal to or above the predetermined threshold, from the image of the high-reflectance wavelength as the inclusion area. Thereafter, based on the amount of the light having the low-reflectance wavelength of an area of the image of the low-reflectance wavelength, which corresponds to the inclusion area, the detector 45 detects the detection-target site.

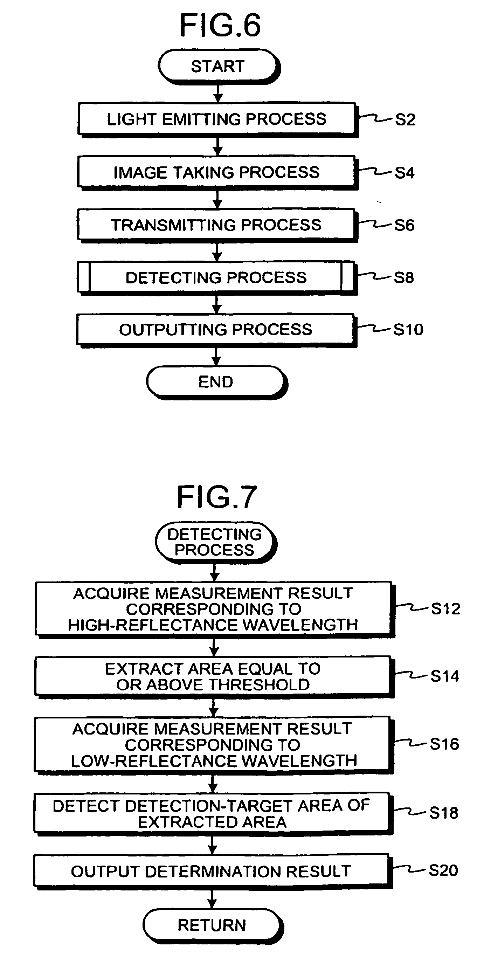

A detecting process of the body-insertable apparatus system, which detects whether there is a detection-target site such as a bleeding site, will be described below with reference to FIG. 6. As shown in FIG. 6, first, the emitter 32 performs a light emitting process for emitting the light having the low-reflectance wavelength and the light having the high-reflectance wavelength (step S2). Subsequently, the imaging device 34 performs an image-taking process for taking an image by receiving the light having the low-reflectance wavelength and the light having the high-reflectance wavelength (step S4). Thereafter, a transmitting process is performed for transmitting the image, which is taken by the imaging device 34, to the workstation 4 through the receiving apparatus 2 and the portable recording medium 5 (step S6). In the workstation 4, the detector 45 performs a detecting process for detecting a detection-target site (step S8). The output unit 47 performs an outputting process for outputting and displaying the image as well as a result of the detection by the detector 45 (step S10).

The detecting process shown in FIG. 6 will be described below with reference to FIG. 7. As shown in FIG. 7, the detector 45 acquires a measurement result corresponding to the high-reflectance wavelength from measurement results about the amount of light of each wavelength, which is received by the imaging device 34 (step S12). In other words, the detector 45 acquires an image of the high-reflectance wavelength. The detector 45 extracts an area, in which the amount of the light having the high-reflectance wavelength is equal to or above the predetermined threshold, from the image of the high-reflectance wavelength as an area that highly likely contains a detection-target site (step S14). This is because it can be determined that an area in which the amount of the light having the high-reflectance wavelength is below the threshold is a portion in the direction of the lumen where no light is received. Subsequently, the detector 45 acquires a measurement result that corresponds to the low-reflectance wavelength from the measurement results acquired by the imaging device 34 (step S16). In other words, the detector 45 acquires an image of the low-reflectance wavelength. The detector 45 detects a detection-target site based on the amount of the light having the low-reflectance wavelength of the area, which is extracted at step S14, from the image of the low-reflectance wavelength (step S18). The detector 45 outputs the image, on which detection is performed, in association with a result of the detection (step S20).

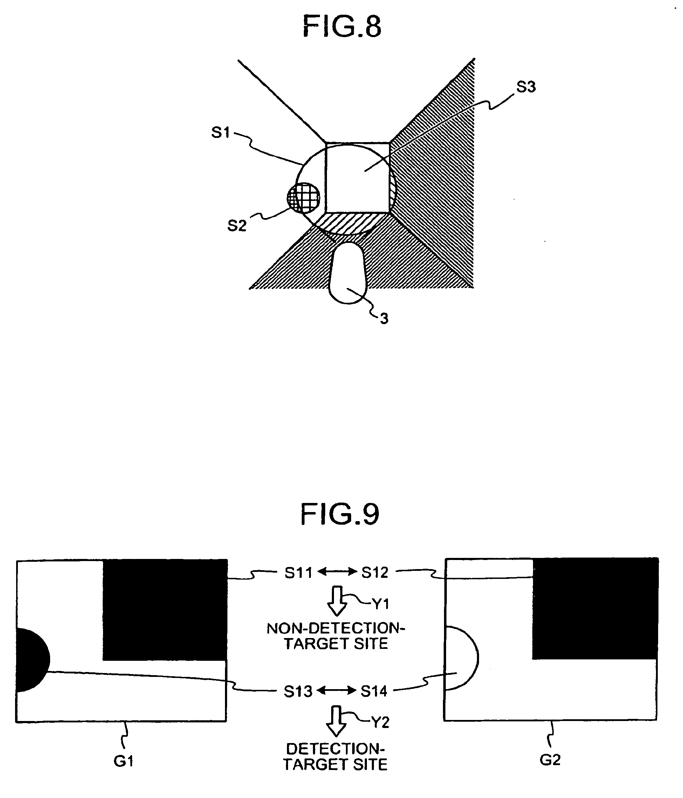

The detecting process of the detector 45 will be described below, taking as an example the case where the capsule endoscope 3 takes an image of an area S1 in the subject 1 as shown in FIG.8. The area S1 includes an area S2 that includes a bleeding site, which is a detection-target site, and an area S3 that corresponds to the direction of the lumen distant from a measurement position.

An image G1 of the low-reflectance wavelength, which is shown on the left in FIG. 9, and an image G2 of a high-reflectance wavelength, which is shown on the right in FIG. 9, are acquired as images of the area S1. The detector 45 detects the detection-target site based on the image G1 and the image G2. At step S12 shown in FIG. 9, the detector 45 determines that the area S12, in which the amount of the light having the high-reflectance wavelength is equal to or below the threshold, of the image G2 is a non-detection-target site that is an area in the direction of the lumen on which no light is reflected and in which no detection target is present, as indicated by an arrow Y1. Therefore, the detector 45 excludes the area S12 from extraction targets. Thereafter, the detector 45 extracts areas other than the area S12 from the image G2 as an inclusion area that may include a detection-target site. At step S12, an area taking the direction of the lumen can be excluded, and also an area in which a site having optical characteristics different from those of a detection-target site is present can be excluded. The detector 45 may compare the image G2 and the image G1 to detect that an area that is dark in both of the image G2 and the image G1 is a non-detection-target site.

The detector 45 determines whether there is a dark area, in which the amount of the light having the low-reflectance wavelength is small, in areas of the image G1 other than the area S11 corresponding to the area S12 of the image G2 detected at step S12. In an area S13 in the image G1, the amount of the light having the low-reflectance wavelength is small. Thus, the area S13 is dark. If a detection-target site is a bleeding site in the area S13, it can be presumed that the applied light having the low-reflectance wavelength is absorbed in hemoglobin of blood; therefore, the light is not reflected. Thus, the detector 45 detects that there is a detection-target site in the area S13, as indicated by an arrow Y2 shown in FIG. 9. The detector 45 may determine that there is a detection-target site in an area that is bright in the image G2 and is dark in the image G1 by comparing the image G2 and the image G1. The detector 45 detects that only images in the direction of a lumen are successively taken, when there is no area, in which the amount of light exceeds the threshold, in each image of the high-reflectance wavelength, i.e., when dark images of the high reflectance wavelength are successive and dark images of the low-reflectance wavelength are successive. Furthermore, the detector 45 detects that the amount of light from the light emitter 32 is insufficient and therefore the images are dark, if dark images of the high-reflectance wavelength and dark images of the low-reflectance wavelength are successive and no image of the high-reflection wavelength appears that contains a bright area.

As described above, the body-insertable apparatus system according to the first embodiment emits the light having the low-reflectance wavelength, which is used for making a detection-target site, such as a bleeding site, conspicuous, as well as the light having the high-reflectance wavelength with the high reflectance with respect to a detection-target site. Based on the amount of the light having the high-reflectance wavelength, the body-insertable apparatus system detects the detection-target site. Accordingly, in the first embodiment, the reason why an area in an image of the low-reflectance wavelength is displayed dark can be distinguished between the following causes: absorption by hemoglobin in blood, the viewing direction is a direction of a lumen, or the amount of light is insufficient. This makes it possible to accurately detect a bleeding site or a tumor site, which is a detection-target site, compared with conventional techniques.

To acquire the amount of light having the low-reflectance wavelength and the amount of light having the high-reflectance wavelength, it suffices that one of the light emitter 32 and the imaging device 34 emits or receives the light having the low-reflectance wavelength and the light having the high-reflectance wavelength. For example, the light emitter 32 may include an LED that emits the low-reflectance wavelength and an LED that emits the high-reflectance wavelength and the imaging device 34 may receive the low-reflectance wavelength and the high-reflectance wavelength as well as a light of a different wavelength range. The light emitter 32 may include, in addition to a white-light LED, a filter that allows only the low-reflectance wavelength to pass through and a filter that allows only the high-reflectance wavelength to pass through to emit the light having the low-reflectance wavelength and the light having the high-reflectance wavelength. The imaging device 34 may include a filter that allows only the low-reflectance wavelength to pass through and a filter that allows only the high-reflectance wavelength to pass through to receive the light having the low-reflectance wavelength and the light having the high-reflectance wavelength. Of course, both of the light emitter 32 and the imaging device 34 can be configured to emit and receive the light having the low-reflectance wavelength and the light having the high-reflectance wavelength. This increases accuracy in receiving the light having the low-reflectance wavelength and the light having the high-reflectance wavelength to increase the accuracy of the detecting process.

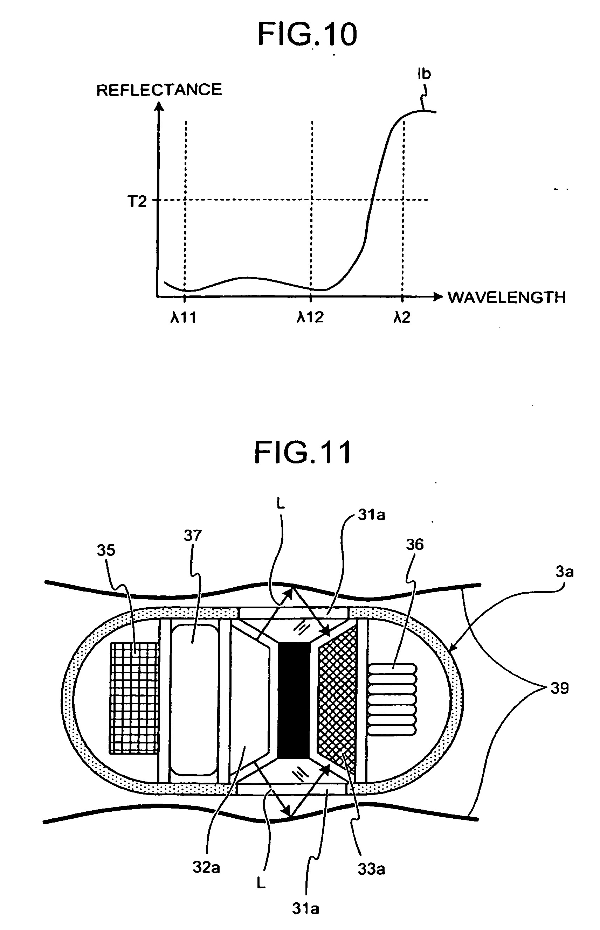

In the first embodiment, conventional LEDs and an imaging device can be used. Therefore, although wavelengths from 415 nm to 580 nm are described as the low-reflectance wavelength, it suffices that the low-reflectance wavelength is a wavelength in a bandwith having a low reflectance with respect to a detection-target site. For example, the low-reflectance wavelength is not limited to the blue light and the green light. As shown in FIG. 10, the low-reflectance wavelength may be a wavelength λ11 with a low reflectance with respect to a bleeding site, which is the bandwidth of ultraviolet rays, or a wavelength λ12 that is a yellow light. It suffices that the threshold used in the detecting process of the detector 45 is set based on the reflectance of the light having the low-reflectance wavelengths λ11, λ12 in the curve 1b, the reflectance of the light having the high-reflectance wavelength λ2 in the curve lb, and the value T2 obtained in consideration for diffuse reflection and measurement errors. Because conventional LEDs and imaging devices can be used, although wavelengths from 615 nm to 635 nm are described above as the high-reflectance wavelength, it suffices that the high-reflectance wavelength is a wavelength with a high reflectance with respect to a detection-target site and is may be, for example, a wavelength of the bandwidth of infrared light.

In the first embodiment, the capsule endoscope 3 that is provided with the transparent cover at the end portion and performs the image-taking process in the longitudinal direction of the capsule endoscope 3 is taken as an example and a description is made. Alternatively, as shown in FIG. 11, a side-view type capsule endoscope 3a may be used that is provided with a light emitter 32a and a light receiver 33a on its side surface and performs an image-taking process in the lateral direction of the capsule endoscope. A light L that is emitted from the light emitter 32a is reflected on, for example, an intestine wall 39 and received by the light receiver 33a through a transparent cover 31a provided on the side surface of the capsule endoscope 3a. Using the side-view type capsule endoscope 3a shown in FIG. 11 shortens the distance between the capsule endoscope 3a and the interior of the subject 1, which makes it possible to perform stable detection with large measurement intensity. It is considered that the capsule endoscope 3a is useful, especially when the distance between the capsule endoscope 3a and the subject becomes large, for example, when the capsule endoscope 3a enters a large lumen or when the direction of the capsule endoscope 3a deviates in the subject 1, because the capsule endoscope 3a can detect whether there is a detection-target site.

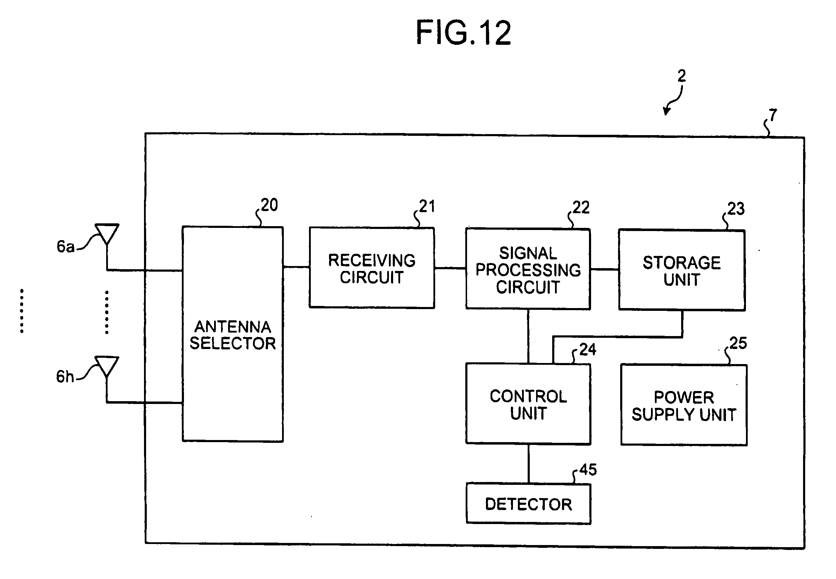

In the first embodiment, the case where the workstation 4 includes the detector 45 is described. However, the detector 45 is not limited to this case. Alternatively, for example, the external device 7 forming the receiving apparatus 2 may include the detector 45 as shown in FIG. 12. The external device 7 includes, in addition to the detector 45, an antenna selector 20 that selects an antenna suitable to receive from the receiving antennas 6a to 6h; a receiving circuit 21 that performs the demodulating process on a wireless signal, which is received through a receiving antenna; a signal processing circuit 22 that processes the signal, which is output from the receiving circuit 21, and that outputs image information; a storage unit 23 that stores therein the image, which is processed by the signal processing circuit 22; a control unit 24 that controls each unit; and a power supply unit 25 that supplies a drive power to each unit. The detector 45 detects whether there is a detection-target site based on image information of the low-reflectance wavelength and the high-reflectance wavelength, which is output from the signal processing circuit 22. The control unit 24 stores a result of the detection in the storage unit 23 in association with the image information.

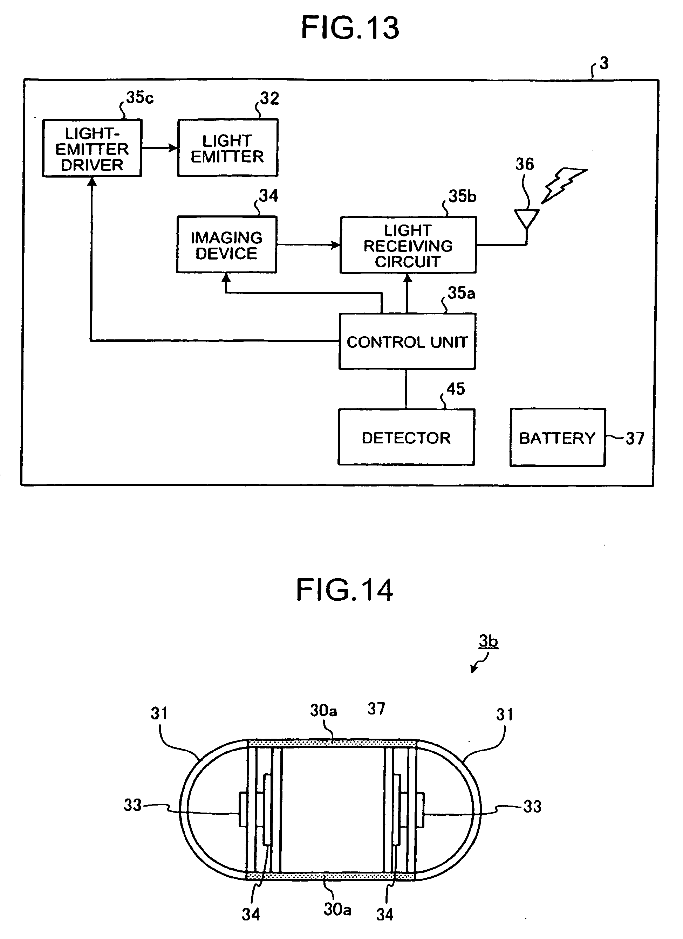

Also, the capsule endoscope 3 may include the detector 45 as shown in FIG. 13. The capsule endoscope 3 includes, in addition to the detector 45, the light emitter 32; the imaging device 34; the battery 37; a control unit 35a that controls each unit; a light receiving circuit 35b that modulates information of an image taken by the imaging device 34 to generate a wireless signal; a light-emitter driver 35c that controls driving of the light emitter 32; and an antenna 36 that wirelessly transmits the signal, which is output from the light receiving circuit 35b. The control unit 35a, the light receiving circuit 35b, and the light-emitter driver 35c are provided in the processing circuit 35. The detector 45 detects a detection-target site by acquiring the amounts of the light having the low-reflectance wavelength and the light having the high-reflectance wavelength of an area of the image taken by the imaging device 34, which corresponds to a detection-target area. The control unit 35a transmits a result of the detection in association with the wireless signal, which is generated by the light receiving circuit 35b, to the receiving apparatus 2 via the antenna 36.

In the first embodiment, a monocular capsule endoscope is described as an example of the capsule endoscope 3. Alternatively, a multinocular capsule endoscope 3b may be used that is provided with transparent covers 31 on both left and right end portions in FIG. 14, and plural light emitters 32, lenses 33, and imaging devices 34 are provided such that they correspond to the left and right end portions, as shown in FIG. 14. In the capsule endoscope 3b, the two imaging devices 34 face in opposite directions and therefore take images in the respective opposite directions. Because the capsule endoscope 3b can take in-vivo images of the body cavity with the imaging devices 34, a large area can be simultaneously observed compared with the monocular capsule endoscope 3. This leads to an effect that observation performance improves. Specifically, because the imaging devices 34 of the capsule endoscope 3b face in the opposite directions, images of front and rear areas of the capsule endoscope 3b can be simultaneously taken and a larger area can be efficiently observed. The ends of the capsule endoscope 3b shown on the left and right in the figure are covered with the transparent covers, and thus the units forming the capsule endoscope 3b are incorporated in a cylindrical housing 30a. The processing circuit 35, the antenna 36, and the battery 37, which are shown in FIG. 2, are omitted in FIG. 14.

### (Second embodiment)

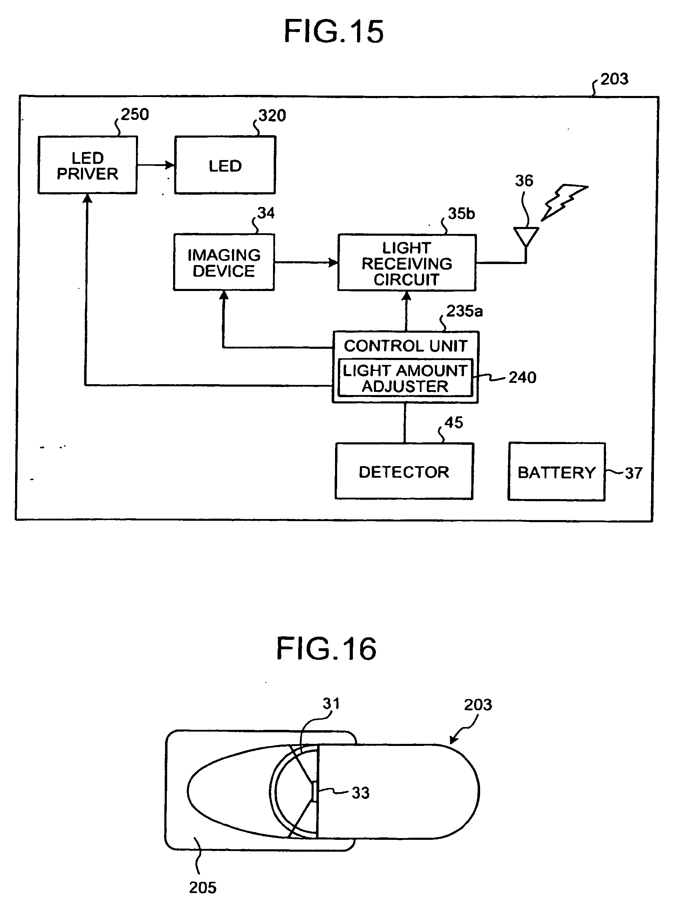

Subsequently, a second embodiment will be described. In the second embodiment, an amount of electric power that is consumed by a capsule endoscope is reduced by adjusting an amount of electric power that is supplied to each light emitting device forming a light emitter of the capsule endoscope. FIG. 15 is a block diagram showing a configuration of the capsule endoscope according to the second embodiment. As shown in FIG. 15, a capsule endoscope 203 according to the second embodiment includes an LED driver 250 instead of the light-emitter driver 35c of the capsule endoscope 3 shown in FIG. 13, and includes a control unit 235a instead of the control unit 35a of the capsule endoscope 3. An LED 320 that forms a light emitter 32 is an LED group formed by plural LEDs that emit lights of predetermined wavebands. The LED driver 250 supplies electric power from the battery 37 to each LED to cause each LED to emit light. The control unit 235a has the same functions as those of the control unit 35a. The control unit 235a includes a light amount adjuster 240 that controls an amount of electric power, which is supplied by the LED driver 250 to each LED of the LED 320.

To adjust the amount of electric power to be supplied to each LED, as shown in FIG. 16, in the state where an end portion of the capsule endoscope 203 is equipped with a cylindrical cap 205 whose interior is white, a light of a predetermined output is emitted by the LED 320 and the light reflected on the interior of the cap 205 is focused on a lens 33, so that the light is received by the imaging device 34. The light amount adjuster 240 acquires a white balance, which is a ratio of measurement values of an imaging device 34 between plural wavelengths to be used. The light amount adjuster 240 calculates an adjustment value, with which an output value of each wavelength is optimized, based on the value of the white balance. In this manner, the light amount adjuster 240 controls the amount of electric power to be supplied to each LED. By controlling the amount of electric power to be supplied to each LED, the light amount adjuster 240 adjusts the amount of light of each wavelength to an amount of light necessary for observing the interior of the subject 1. For example, the amount of electric power to be supplied to an LED that emits a relatively large amount of light having a large wavelength is adjusted such that the amount of light to be emitted is reduced to an appropriate value. Also, the light amount adjuster 240 may control the amount of light to be supplied to each LED, based on a reflectance of a light having a low-reflectance wavelength and a reflectance of a light having a high-reflectance wavelength with respect to a detection-target site. For example, the light amount adjuster 240 controls an amount of power to be supplied to an LED that emits a light having the low-reflectance wavelength, which is used to observe the interior of the subject 1, such that the LED emits an amount of light that assures predetermined accuracy in taking images. The light amount adjuster 240 controls the amount of electric power to be supplied to an LED that emits a light having the high-reflectance wavelength in order to reduce the amount of light to be emitted to a level such that the detector 45 can perform the detecting process, i.e., such that the light having the high-reflectance wavelength and diffuse reflection light, which are reflected on the detection-target site, can be sufficiently distinguished from each other. This reduces power consumption.

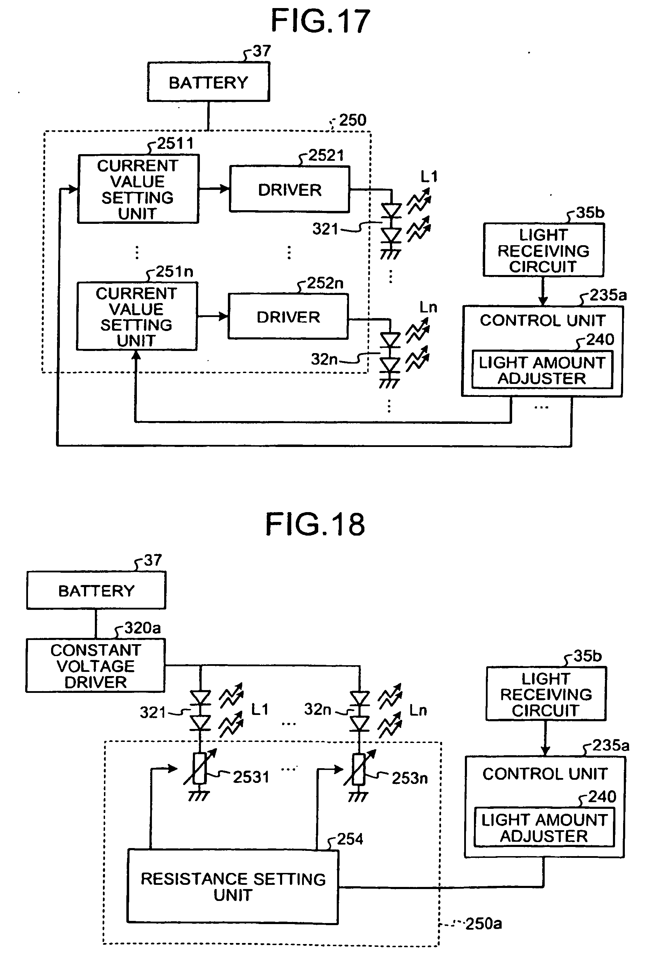

Subsequently, the case where a current value for driving each of the LEDs that form the LED 320 is adjusted will be described as a method of adjusting the amount of electric power to be supplied to the LED 320. FIG. 17 is a block diagram showing a main part of the capsule endoscope 203 shown in FIG. 15. As shown in FIG. 17, the LED driver 250 includes current value setting units 2511 to 251n and drivers 2521 to 252n for each of LEDs 321 to 32n that emits lights L1 to Ln having predetermined wavelengths, respectively. The current value setting units 2511 to 251n adjust the amounts of current to be supplied to the LEDs 321 to 32n, to which the current value setting units 2511 to 251n are connected through the drivers 2521 to 252n, based on the adjustment value of the light amount adjuster 240. The drivers 2521 to 252n supplies the current values, which are set by the current value setting units 2511 to 251n, to the LEDs 321 to 32n to which the drivers 2521 to 252n are respectively connected. Accordingly, the LEDs 321 to 32n emit lights in the amounts corresponding to the amounts of power that are supplied. The LED driver 250 controls the amounts of current to be supplied to each of the LEDs 321 to 32n by controlling the current value setting units 2511 to 251n and the drivers 2521 to 252n.

As described above, in the second embodiment, by adjusting the amount of electric power supplied to the LEDs that emit lights having the respective wavelengths, the power consumption of the capsule endoscope 203 with a limited battery capacity can be reduced. Furthermore, in the second embodiment, by reducing the electric power necessary for emitting a light having the high-reflectance wavelength can be reduced to a level such that the detector 45 can perform the detecting process, an increase of the power consumption, which is caused because the light having the high-reflectance wavelength is emitted, can be reduced.

As shown in FIG. 18, the amounts of electric power to be supplied to the LEDs may be controlled with respect to a driver that supplies a predetermined voltage by increasing or reducing a resistance for adjusting the amount of current. The LEDs 321 to 32n are connected to a constant voltage driver 320a that applies a predetermined voltage. An LED driver 250a includes resistors 2531 to 253n that are connected in series to the LEDs 321 to 32n, and that has variable resistance values; and a resistance setting unit 254 that adjusts the resistance values of the resistors 2531 to 253n based on the white balance adjustment value of the light amount adjuster 240. The resistors 2531 to 253n and the resistance setting unit 254 are embodied by, for example, an electric volume controller that is a resistor for adjusting the amount of current. In this manner, the amounts of current to be supplied to the LEDs 32a to 32n may be adjusted by adjusting the resistance values of the LEDs 321 to 32n.

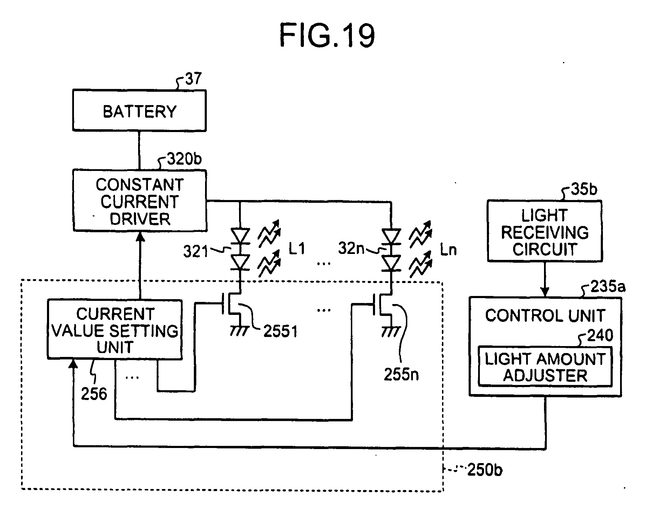

The LED driver 250 may be replaced by an LED driver 250b that includes switches 2551 to 255n and a current value setting unit 256 as shown in FIG. 19. The switches 2551 to 255n switch between on and off states of the LEDs 321 to 32n, to which the switches 2551 to 255n are connected, respectively, under the control of the current value setting unit 256. The current value setting unit 256 controls the amount of current of a constant current driver 320b, which is to be supplied to each of the LEDs 321 to 32n. The constant current driver 320b is connected to the switches 2551 to 255n and the LEDs 321 to 32n. The current value setting unit 256 can select whether to supply electric power to each of the LEDs 321 to 32n by controlling the switches 2551 to 255n. In addition, the current value setting unit 256 can select an amount of electric power to be supplied to each of the LEDs 321 to 32n by controlling the value of the current that is supplied by the constant current driver 320b.

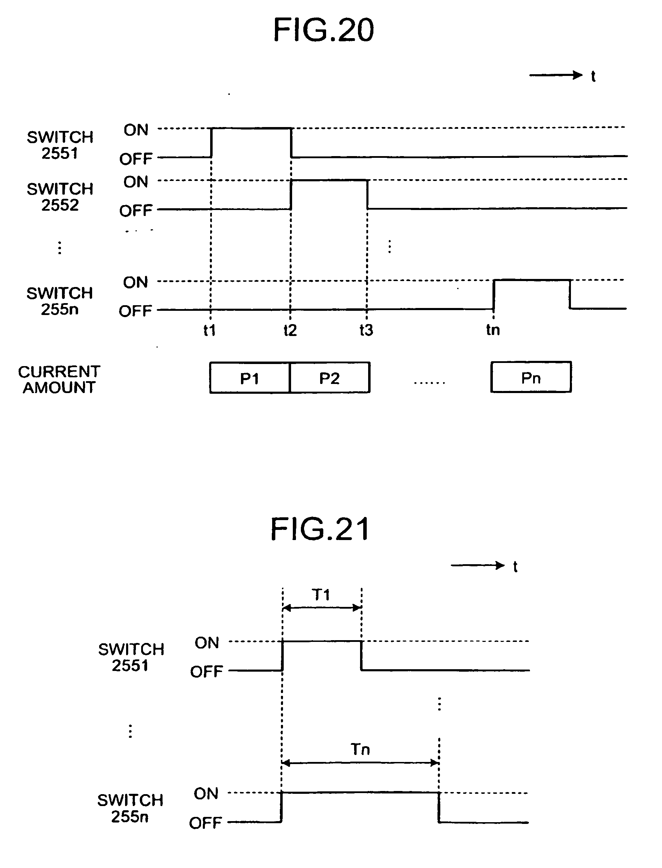

For example, the current value setting unit 256 supplies electric power to the LEDs 321 to 32n in a time-division manner. Specifically, as shown in FIG. 20, the switches 2551 to 255n are turned on in the time-division manner to control the time during which each of the LEDs 321 to 32n emits a light. This reduces the power consumption of the capsule endoscope 203. For example, the current value setting unit 256 turns on the switch 2551, which is connected to the LED 321, from a time t1 to a time t2. In this case, the current value setting unit 256 adjusts a current value of the constant current driver 320b to a current value P1 that corresponds to an adjustment value for the LED 321, which is obtained by the light amount adjuster 240. As a result, the LED 321 emits an amount of light that corresponds to the adjustment value of the light amount adjuster 240 from the time t1 to the time t2. The current value setting unit 256 turns on the switch 2552, which is connected to an LED 322, from the time t2 to a time t3, and adjusts the current value of the constant current driver 320b to a current value P2 that corresponds to an adjustment value for the LED 322, which is obtained by the light amount adjuster 240. As a result, the LED 322 emits an amount of light that corresponds to the adjustment value of the light amount adjuster 240 from the time t2 to the time t3.

The current value setting unit 256 may change a period during which the LEDs 321 to 32n are provided with electric power for the respective LEDs 321 to 32n. Specifically, as shown in FIG. 21, by turning on the switches 2551 to 255n during the periods corresponding respectively to the LEDs 321 to 32n, the time during which each of the LEDs emits a light is controlled. This reduces the power consumption of the capsule endoscope 203. For example, the current value setting unit 256 turns on the switch 2551, which is connected to the LED 321, during a period T1, and adjusts the current value of the constant current driver 320b to a current value corresponding to the adjustment value for the LED 321, which is obtained by the light amount adjuster 240. During a period Tn, the switch 255n, which is connected to the LED 32n, is turned on, and the current value of the constant current driver 320b is adjusted to a current value that corresponds to a white balance adjustment value for the LED 32n, which is obtained by the light amount adjuster 240. For example, when the capsule endoscope 203 moves forward through a cavity having a small inner diameter, a variation in the direction of movement of the capsule endoscope 203 is small. Therefore, it suffices that the detector 45 performs detection using an image of the high-reflectance wavelength at predetermined intervals. In this case, the LED 321 that emits a light having a high reflectance wavelength does not need to emit a light having the high-reflectance wavelength at all times. It suffices that, for example, the LED 321 emits an amount of light having the high-reflectance wavelength with which the detector 45 can perform the detecting process during the period T1 shown in FIG. 21. In contrast, the LED 32n that emits a light having the low-reflectance wavelength emits an amount of light having the low-reflectance wavelength that is sufficient to observe the interior of the subject 1 during a period Tn necessary for observing the interior of the subject 1. In this manner, the light amount adjuster 240 may control the amount of power, which is to be supplied, by setting the time during which a light is emitted and the amount of light to be emitted with respect to each of the LEDs 321 to 32n.

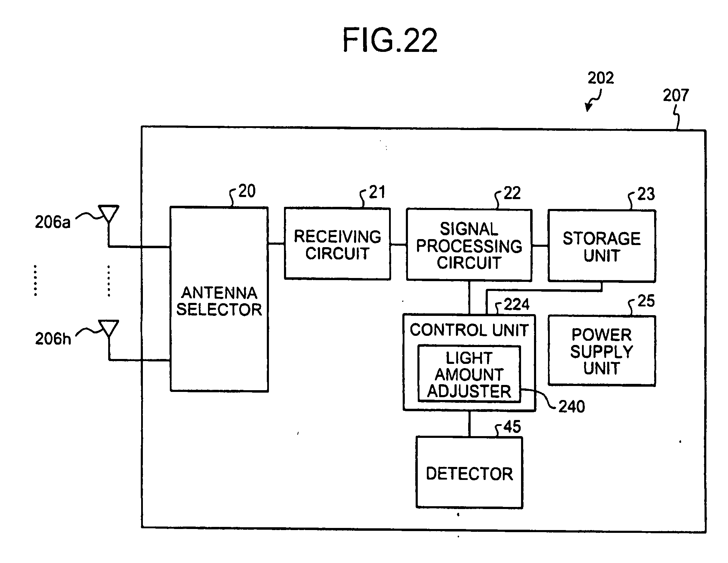

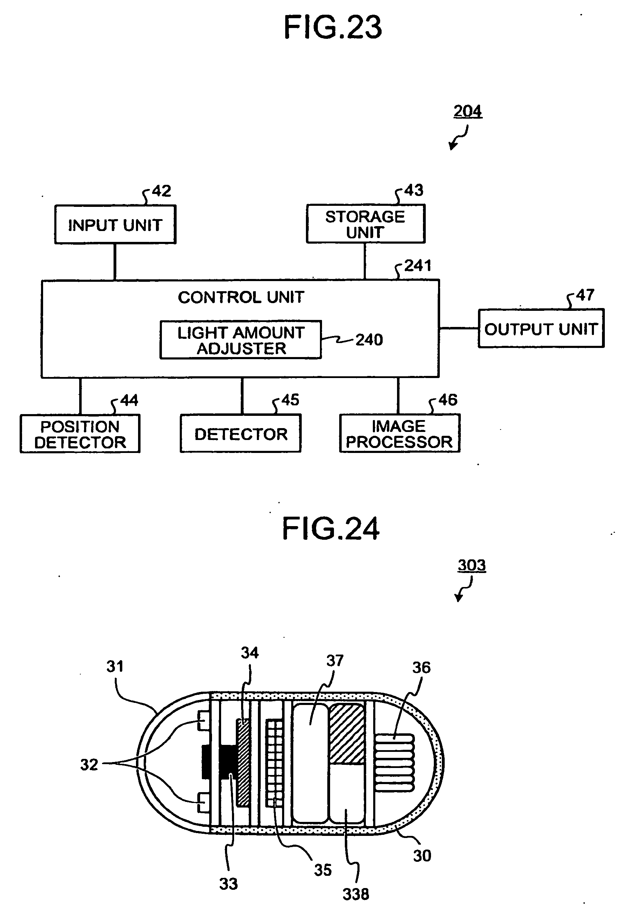

In the second embodiment, the case where the light amount adjuster 240 is provided in the capsule endoscope 203 is described. However, the light amount adjuster 240 is not limited to this case. For example, in the case of a body-insertable apparatus system that enables bidirectional communications, the light amount adjuster 240 may be provided in a control unit 224 of an external device 207 of the receiving apparatus 202 as shown in FIG. 22. In this case, the light amount adjuster 240 calculates an adjustment value for each LED based on measurement results that are obtained when the cap 205 is equipped and received through the antennas 206a to 206h from the capsule endoscope. The receiving apparatus 202 transmits the adjustment values for the respective LEDs to the capsule endoscope through antennas 206a to 206h. In the capsule endoscope, the LED drivers 250, 250a, or 250n adjusts the electric power supplied to the LEDs 321 to 32n based on the received white balance adjustment values in order to control the amounts of light to be emitted. As shown in FIG. 23, the light amount adjuster 240 may be provided in a control unit 241 of a workstation 204. In this case, adjustment values to the LEDs are calculated based on the measurement results, which are obtained when the cap 205 is equipped and received from the capsule endoscope through the receiving apparatus. The workstation 204 transmits the adjustment values for the respective LEDs to the capsule endoscope through the receiving apparatus.

### (Third embodiment)

Subsequently, a third embodiment will be described. In the third embodiment, a magnetically-guiding system is combined with the body-insertable apparatus system according to the first or second embodiment. When a detection-target site is found, the capsule endoscope is guided by applying a magnetic field from the outside of the capsule endoscope to turn the capsule endoscope toward the position of the detection-target site or move the capsule endoscope close to the detection-target site. In the third embodiment, the magnetically-guiding system is applied to the capsule endoscope according to the first embodiment from the capsule endoscopes of the first and second embodiments.

FIG. 24 is a schematic diagram showing a configuration of a capsule endoscope according to the third embodiment. As shown in FIG. 24, a capsule endoscope 303 according to the third embodiment is configured to further include a permanent magnet 338 for guidance, which is provided inside, compared with the capsule endoscope 3 shown in FIG. 2. When a magnetic field is applied from the outside, the permanent magnet 338 is turned according to the direction of the magnetic field. Along with the change in the posture of the permanent magnet 338, the capsule endoscope 303 is turned as well. When a magnetic field is applied from the outside, the position of the permanent magnet 338 is changed due to the magnetic field, and the permanent magnet 338 moves. Thus, along with the change in the position of the permanent magnet 338, the position of the capsule endoscope 303 is changed and the capsule endoscope 303 moves forward as well.

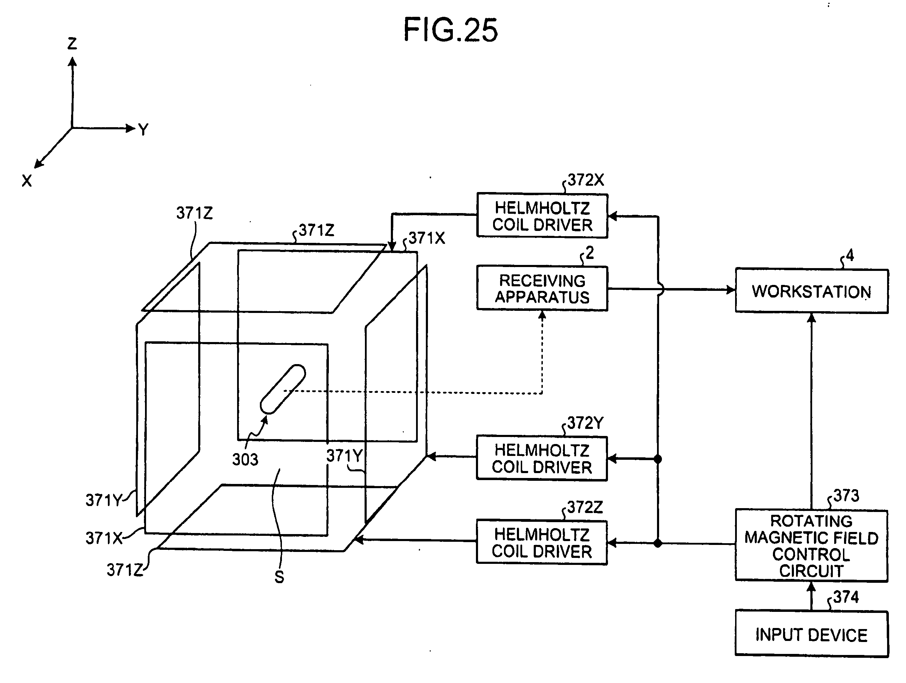

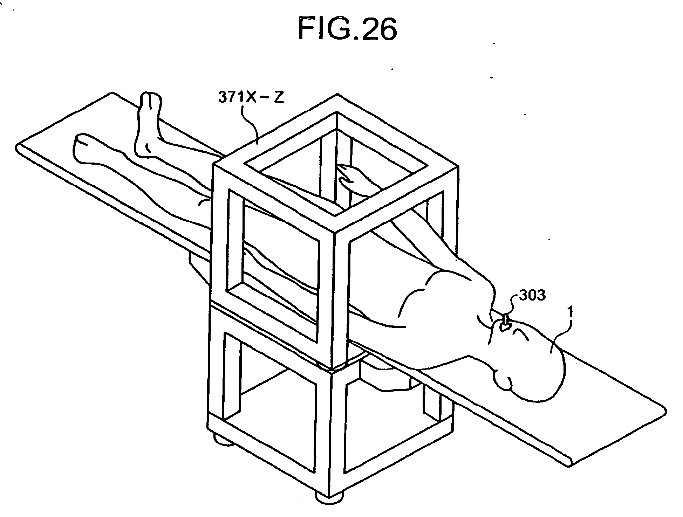

A schematic configuration of a body-insertable apparatus system according to the third embodiment will be described below. FIG. 25 is a schematic diagram of the body-insertable apparatus system according to the third embodiment. FIG. 26 is a diagram for describing the body-insertable apparatus system shown in FIG. 25.

As shown in FIG. 25, the body-insertable apparatus system according to the third embodiment further includes Helmholtz coil units 371X, 371Y, and 371Z that are arranged outside an operation area of the capsule endoscope 303; and Helmholtz coil drivers 372X, 372Y, and 372Z that control and amplify currents that are respectively supplied to the Helmholtz coil units 371X, 371Y, and 371Z, compared with the body-insertable apparatus system shown in FIG. 1. The Helmholtz coil units 371X, 371Y, and 371Z generate parallel magnetic fields that drive the capsule endoscope 303 in X, Y, and Z directions. In other words, the Helmholtz coil units 371X, 371Y, and 371Z generate guidance magnetic fields that are applied to the permanent magnet 338 of the capsule endoscope 303.

The Helmholtz coil units 371X, 371Y, and 371Z are formed to be approximately rectangular as shown in FIG. 26. The Helmholtz coil units 371X, 371Y, and 371Z include three pairs of the Helmholtz coil units 371X, 371Y, and 371Z that are opposed to each other. Each of the pairs of the Helmholtz coil units 371X, 371Y, and 371Z are arranged to be approximately perpendicular to X, Y, and Z axes shown in FIG. 25. The Helmholtz coil unit 371X is arranged to be approximately perpendicular to the X-axis. The Helmholtz coil unit 371Y is arranged to be approximately perpendicular to the Y-axis. The Helmholtz coil unit 371Z is arranged to be approximately perpendicular to the Z-axis. The Helmholtz coil driver 372X controls the Helmholtz coil unit 371X. The Helmholtz coil driver 372Y controls the Helmholtz coil unit 371Y. The Helmholtz coil driver 372Z controls the Helmholtz coil unit 371Z.

The Helmholtz coil units 371X, 371Y, and 371Z are arranged such that a space approximately in the form of a rectangular parallelepiped is formed inside. The space S serves as an operation space of the capsule endoscope 303 as shown in FIG. 25 and also serves as a space in which the subject 1 is arranged as shown in FIG. 26.

The body-insertable apparatus system according to the third embodiment includes a rotating magnetic field control circuit 373 that controls directions of the parallel magnetic fields, which are guidance magnetic fields that drive the capsule endoscope 303; and an input device 374 that outputs a moving direction of the capsule endoscope 303, which is input by an input operation of a practitioner, to the rotating magnetic field control circuit 373.

In the body-insertable apparatus system, the rotating magnetic field control circuit 373 controls the directions of the parallel magnetic fields. The Helmholtz coil drivers 372X, 372Y, and 372Z generate the parallel magnetic fields, which are controlled respectively by the rotating magnetic field control circuit 373, in the Helmholtz coil units 371X, 371Y, and 371Z. As a result, the posture of the permanent magnet 338 mounted on the capsule endoscope 303 is changed according to the parallel magnetic fields. Along with the change, the posture and the moving direction of the capsule endoscope 303 are changed as well.

In the third embodiment, as described above, by controlling the directions of the magnetic fields that are applied to the permanent magnet mounted on the capsule endoscope 303, the direction in which force is applied to the magnet is controlled and the posture and the moving direction of the capsule endoscope 303 are controlled. In the third embodiment, when a detection-target site is found, the capsule endoscope can be turned toward a position of a detection-target site or the capsule endoscope can be moved close to the detection-target site, which makes it possible to observe the detection-target site well.

The third embodiment is described taking as an example the Helmholtz coil units 371X, 371Y, and 371Z based on the three axes. However, the coils may be the one that does not necessarily satisfy the conditions of Helmholtz coil. The coils may be, for example, circular or approximately rectangular. In addition, the intervals between the coils may be out of the conditions of Helmholtz coil as long as the functions of the third embodiment are satisfied.

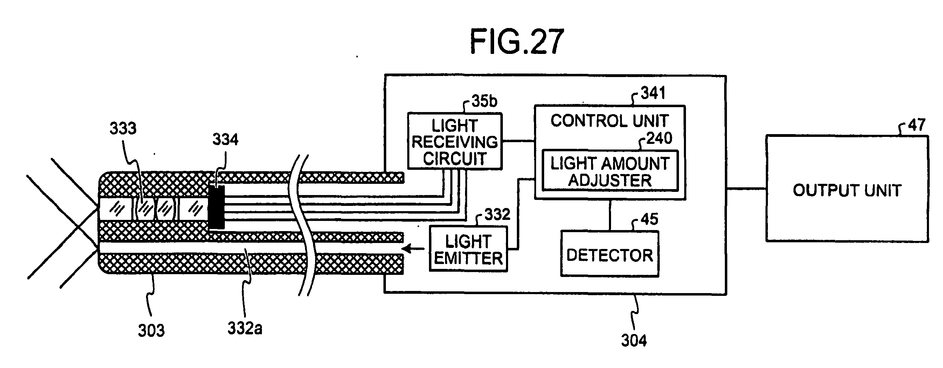

Although the case where the capsule endoscope is inserted into the subject 1 is described in the first to third embodiments, it is also applicable to the case where an insertion unit 303 that includes a transmission path 332a for transmitting a light from a light emitter 332; a lens system 333; and an imaging device 334, and that is connected to a work station 304 with a wireline is inserted into the subject 1 as shown in FIG. 27. In this case, inclusion of the detector 45 and the light amount adjuster 240 in a control unit 341 makes it possible to accurately detect a bleeding site or a tumor site, which is a detection-target site, and reduce the power consumption.

### INDUSTRIAL APPLICABILITY

As described above, the present invention is useful for a body-insertable apparatus system that makes a detection-target site conspicuous while the detection-target site is displayed, and is suitable to achieve accurate detection of sites to be detected and power saving.

## Claims

1. A body-insertable apparatus system for taking in-vivo images (G1, G2) of a subject (1), comprising:
a capsule endoscope (3; 3a; 3b; 203; 303) and a detector (45), wherein:
the capsule endoscope includes:
a light emitting unit (32; 32a) configured to emit a light having a first wavelength and a light having a second wavelength, wherein:
a detection-target site to be detected in the subject (1) has a first reflectance for the first wavelength and a second reflectance higher than the first reflectance for the second wavelength, and
an area (S1) including the detection-target site is illuminated by the light emitting unit (32; 32a); and
an imaging unit (34) configured to generate a first in-vivo image (G1) by receiving the light having the first wavelength and generate a second in-vivo image (G2) by receiving the light having the second
wavelength, and
the detector (45) is configured to determine that there is a detection-target site in an area that is bright in the second in-vivo image (G2) and is dark in the first in-vivo image (G1).

2. The body-insertable apparatus system according to claim 1, wherein a threshold is set based on the first reflectance and the second reflectance.

3. The body-insertable apparatus system according to claim 1 or 2, wherein the detection-target site is a bleeding site.

4. The body-insertable apparatus system according to any one of claims 1 to 3, wherein
the first wavelength is in a range of 415 nm to 580 nm, and
the second wavelength is in a range of 615 nm to 635 nm.

5. The body-insertable apparatus system according to any one of claims 1 to 4, further comprising:
an image processing unit (46) configured to generate a detection image including the detection-target site from the in-vivo images (G1, G2) so that the detection-target site is enhanced in the detection image; and
an image display unit (47) configured to display information that contains the detection image.

6. The body-insertable apparatus system according to any one of claims 1 to 5, wherein
the light emitting unit (32; 32a) includes at least
a first light emitting device configured to emit the light having the first wavelength; and
a second light emitting device configured to emit the light having the second wavelength.

7. The body-insertable apparatus system according to claim 6, wherein the first and second light emitting devices are LEDs.

## Patentansprüche

1. Körpereinführbares Vorrichtungssystem zur Aufnahme von In-vivo-Bildern (G1, G2) eines Subjekts (1), umfassend:
ein Kapselendoskop (3; 3a; 3b; 203; 303) und eine Erfassungseinrichtung (45),
wobei:
das Kapselendoskop umfasst:
eine lichtemittierende Einheit (32; 32a), die konfiguriert ist, um Licht mit einer ersten Wellenlänge und Licht mit einer zweiten Wellenlänge zu emittieren, wobei:
eine Erfassungszielstelle, die in dem Subjekt (1) zu erfassen ist, eine erste Reflexion für die erste Wellenlänge und eine zweite Reflexion für die zweite Wellenlänge aufweist, die höher als die erste Reflexion ist, und
ein Bereich (S1), der die Erfassungszielstelle umfasst, durch die lichtemittierende Einheit (32; 32a) beleuchtet wird; und
eine Bildeinheit (34), die konfiguriert ist, um ein erstes In-vivo-Bild (G1) durch Empfangen des Lichts mit der ersten Wellenlänge zu erzeugen, und um ein zweites In-vivo-Bild (G2) durch Empfangen des Lichts mit der zweiten Wellenlänge zu erzeugen; und
die Erfassungseinrichtung (45) konfiguriert ist, um zu bestimmen, dass eine Erfassungszielstelle in einem Bereich vorliegt, der in dem zweiten In-vivo-Bild (G2) hell und in dem ersten In-vivo-Bild (G1) dunkel ist.

2. Körpereinführbares Vorrichtungssystem gemäß Anspruch 1, wobei eine Schwelle auf der Grundlage der ersten Reflexion und der zweiten Reflexion gesetzt ist.

3. Körpereinführbares Vorrichtungssystem gemäß Anspruch 1 oder 2, wobei die Erfassungszielstelle eine Blutungsstelle ist.

4. Körpereinführbares Vorrichtungssystem gemäß zumindest einem der Ansprüche 1 bis 3, wobei
die erste Wellenlänge in einem Bereich von 415 nm bis 580 nm liegt, und
die zweite Wellenlänge in einem Bereich von 615 nm bis 635 nm liegt.

5. Körpereinführbares Vorrichtungssystem gemäß zumindest einem der Ansprüche 1 bis 4, weiterhin umfassend:
eine Bildverarbeitungseinheit (46), die konfiguriert ist, um ein Erfassungsbild einschließlich der Erfassungszielstelle aus den In-vivo-Bildern (G1, G2) zu erzeugen, so dass die Erfassungszielstelle in dem Erfassungsbild gesteigert ist; und
eine Bildanzeigeeinheit (47), die konfiguriert ist, um Informationen anzuzeigen, die das Erfassungsbild enthalten.

6. Körpereinführbares Vorrichtungssystem gemäß zumindest einem der Ansprüche 1 bis 5, wobei
die lichtemittierende Einheit (32; 32a) zumindest umfasst
eine erste lichtemittierende Einrichtung, die konfiguriert ist, um das Licht mit der ersten Wellenlänge zu emittieren; und
eine zweite lichtemittierende Einrichtung, die konfiguriert ist, um das Licht mit der zweiten Wellenlänge zu emittieren.

7. Körpereinführbares Vorrichtungssystem gemäß Anspruch 6, wobei die erste und zweite lichtemittierende Einrichtung LEDs sind.

## Revendications

1. Système d'appareil insérable dans un corps pour prendre des images in vivo (G1, G2) d'un sujet (1), comprenant :
un endoscope de type capsule (3 ; 3a ; 3b ; 203 ; 303) et un détecteur (45), dans lequel :
l'endoscope de type capsule inclut :
une unité électroluminescente (32 ; 32a) configurée pour émettre une lumière ayant une première longueur d'onde et une lumière ayant une deuxième longueur d'onde, dans lequel :
un site de cible de détection devant être détecté dans le sujet (1) a un premier facteur de réflexion pour la première longueur d'onde et un deuxième facteur de réflexion supérieur au premier facteur de réflexion pour la deuxième longueur d'onde, et
une zone (S1) incluant le site de cible de détection est éclairée par l'unité électroluminescente (32 ; 32a) ; et
une unité d'imagerie (34) configurée pour générer une première image in vivo (G1) par réception de la lumière ayant la première longueur d'onde et générer une deuxième image in vivo (G2) par réception de la lumière ayant la deuxième longueur d'onde, et
le détecteur (45) est configuré pour déterminer qu'il y a un site de cible de détection dans une zone qui est lumineuse dans la deuxième image in vivo (G2) et est sombre dans la première image in vivo (G1).

2. Système d'appareil insérable dans un corps selon la revendication 1, dans lequel un seuil est fixé sur la base du premier facteur de réflexion et du deuxième facteur de réflexion.

3. Système d'appareil insérable dans un corps selon la revendication 1 ou 2, dans lequel le site de cible de détection est un site de saignement.

4. Système d'appareil insérable dans un corps selon l'une quelconque des revendications 1 à 3, dans lequel
la première longueur d'onde est dans une plage de 415 nm à 580 nm, et
la deuxième longueur d'onde est dans une plage de 615 nm à 635 nm.

5. Système d'appareil insérable dans un corps selon l'une quelconque des revendications 1 à 4, comprenant en outre :
une unité de traitement d'image (46) configurée pour générer une image de détection incluant le site de cible de détection à partir des images in vivo (G1, G2) de telle manière que le site de cible de détection est amélioré dans l'image de détection ; et
une unité d'affichage d'image (47) configurée pour afficher une information qui contient l'image de détection.

6. Système d'appareil insérable dans un corps selon l'une quelconque des revendications 1 à 5, dans lequel
l'unité électroluminescente (32 ; 32a) inclut au moins
un premier dispositif électroluminescent configuré pour émettre la lumière ayant la première longueur d'onde ; et
un deuxième dispositif électroluminescent configuré pour émettre la lumière ayant la deuxième longueur d'onde.

7. Système d'appareil insérable dans un corps selon la revendication 6, dans lequel les premier et deuxième dispositifs électroluminescents sont des LED.
